# EUROPÄISCHE PATENTSCHRIFT

(11) **EP 1 507 851 B1**
(45) Veröffentlichungstag und Bekanntmachung des Hinweises auf die Patenterteilung: **05.10.2005**
(21) Anmeldenummer: 03732479.5
(22) Anmeldetag: 27.05.2003
(51) Int. Cl.: C12N 7/02, C12N 7/04

(54) **VERFAHREN ZUM ASSEMBLIEREN VON UNTEREINHEITEN ZU KAPSOIDEN**
METHOD FOR ASSEMBLING SUB UNITS INTO CAPSOIDES
PROCEDE D'ASSEMBLAGE DE SOUS-UNITES EN CAPSIDES

(30) Priorität: 29.05.2002 DE 10224111
(43) Veröffentlichungstag der Anmeldung: 23.02.2005
(73) Patentinhaber: responsif GmbH, 91056 Erlangen (DE)
(72) Erfinder: THIES, Michael, 97074 Würzburg (DE)
(74) Vertreter: Gassner, Wolfgang
(86) Internationale Anmeldenummer: PCT/EP2003/005568
(87) Internationale Veröffentlichungsnummer: WO 2003/100042

(56) Entgegenhaltungen:
- HENKE S ET AL: "Polyoma virus capsid proteins as potential carriers of drugs into mammalian cells" PHARMAZIE, VEB VERLAG VOLK UND GESUNDHEIT. BERLIN, DD, Bd. 53, Nr. 3, SUPPL 1, März 1998 (1998-03), Seite 16 XP002225097 ISSN: 0031-7144
- JANSON JC AND RYDEN L (EDITORS): "Protein Purification: Prinicples, High-Resolution Methods, and Applications, 2nd edition" 1998 , JOHN WILEY & SONS, INC. , NEW YORK XP002252487 Seite 29, letzter Absatz -Seite 30, Absatz 2 Seite 124, Absatz 1 - Absatz 2
- SCOPES RK: "Protein Purification: Principles and Practice 2nd edition" 1988 , SPRINGER , NEW YORK XP002252488 Seite 19 Seite 189, Absatz 1
- BRAUN H ET AL: "Oligonucleotide and plasmid DNA packaging into polyoma VP1 virus-like particles expressed in Escherichia coli" BIOTECHNOLOGY AND APPLIED BIOCHEMISTRY, ACADEMIC PRESS, US, Bd. 29, Nr. PART 1, Februar 1999 (1999-02), Seiten 31-43, XP002225098 ISSN: 0885-4513
- STARK C ET AL: "Efficient intracellular delivery of proteins and low molecular weight substances via polyoma virus-like particles (PVLP)." JOURNAL OF PEPTIDE SCIENCE, Bd. 8, Nr. Supplement, 2002, Seite S215 XP009016233 27th European Peptide Symposium;Sorrento, Italy; August 31-September 06, 2002, 2002 ISSN: 1075-2617
- WEI-CHIH OU ET AL: "The major casid protein, VP1, of human JC virus expressed in Escherichia coli is able to self-assemble into a cpsid-like particle and deliver exogenous DNA int human kdney cells" JOURNAL OF GENERAL VIROLOGY, SOCIETY FOR GENERAL MICROBIOLOGY, READING, GB, Bd. 80, 1999, Seiten 39-46, XP002164832 ISSN: 0022-1317

## Beschreibung

Die Erfindung betrifft ein Verfahren zum Assemblieren von Untereinheiten in einer ein Reduktionsmittel enthaltenden Lösung zu Kapsoiden. Kapsoide können als Vehikel zum Transport von Wirkstoffen, insbesondere Nukleinsäuren, ins Innere von Zellen verwendet werden.

Es ist allgemein bekannt, dass Kapsoide bildende Untereinheiten, so genannte Kapsomere, bei Überschreiten einer bestimmten Ionenstärke oder Calciumionenkonzentration der sie umgebenden Lösung Aggregate bilden können. Ferner ist es bekannt, dass die Untereinheiten unter Ausbildung von Disulfidbrücken zwischen den Untereinheiten oxidieren und dabei Aggregate bilden können. Die Untereinheiten aggregieren jedoch nur unter bestimmten Bedingungen zu Kapsoiden.

Aus Chen, X. S., et al., Molecular Cell 5 (2000), Seiten 557 bis 567 ist es bekannt, das Assemblieren von in E. coli exprimiertem Protein L1 des humanen Papilloma-Virus 16 durch Herabsetzen des pH-Werts zu bewirken. Das Protein L1 bildet als Kapsomere Pentamere, von denen sich jeweils 72 bei pH 5,2 zu einen Kapsoid zusammenlagern. Nachteilig ist dabei, dass die gebildeten Kapsoide unregelmäßig sind und dass das Zusammenlagern nicht quantitativ erfolgt, d.h. es bleiben freie Pentamere zurück.

Aus Braun, H., et al., Biotechnol. Appl. Biochem. 29 (1999), Seiten 31 bis 43 ist es bekannt, Kapsoide aus in E. coli rekombinant hergestellten Untereinheiten zu assemblieren. Die Untereinheiten bestehen dabei aus dem Protein VP1 des Polyoma-Virus. Nach Expression werden die VP1-Proteine als Pentamere aus E. coli in einer ein Reduktionsmittel enthaltenden Lösung niedriger Salzkonzentration isoliert und aufbewahrt. Das Reduktionsmittel verhindert dabei eine durch Ausbildung von Disulfidbrücken zwischen den Pentameren bewirkte Bildung irregulärer Aggregate während der Isolierung und Aufbewahrung. Da die Pentamere in Lösungen hoher Ionenstärke ebenfalls zu unregelmäßig geformten Aggregaten assemblieren, verhindert die niedrige Salzkonzentration die Bildung solcher Aggregate. Um ein Assemblieren zu regelmäßigen Kapsoiden, d.h. Kapsoiden mit einer regelmäßigen aus 72 Pentameren aufgebauten Struktur, zu erreichen, wird das Reduktionsmittel langsam entfernt während die Ionenstärke langsam erhöht wird. Beide Prozesse werden gleichzeitig mittels einer Dialyse über einen Zeitraum von 5 bis 7 Tagen durchgeführt. Nachteilig ist bei diesem im Folgenden als Dialyseverfahren bezeichneten Verfahren, dass es sehr zeitaufwändig ist.

Aus der DE 199 30 676 A1 ist es bekannt, eine strukturverändernde Oxidation an Proteinen durch den Einsatz von Thiolreagenzien, wie z. B. 2-Mercaptoethanol oder Cystein, zu unterdrücken. Eine dadurch verminderte Enzymaktivität kann durch Entfernen des 2-Mercaptoethanols durch Dialyse oder Gelfiltration nahezu vollständig wieder hergestellt werden.

Aufgabe der Erfindung ist es, ein Verfahren zum Assemblieren von regelmäßigen Kapsoiden und einen Kit zur Durchführung des Verfahrens bereitzustellen, welches bzw. welcher die Nachteile nach dem Stand der Technik nicht aufweist. Insbesondere soll das Verfahren schneller durchzuführen sein als das Dialyseverfahren.

Diese Aufgabe wird durch die Merkmale der Ansprüche 1 und 17 gelöst. Zweckmäßige Ausgestaltungen ergeben sich aus den Merkmalen der Ansprüche 2 bis 16, 18 und 19.

Erfindungsgemäß ist ein Verfahren zum Assemblieren von Kapsoide bildenden Untereinheiten in einer ein Reduktionsmittel enthaltenden Lösung zu Kapsoiden vorgesehen, wobei zuerst das Reduktionsmittel inaktiviert oder aus der Lösung entfernt wird und dann die Ionenstärke in der Lösung durch Zusatz mindestens eines Salzes zu der Lösung erhöht wird. Die Ionenstärke wird dabei mindestens so weit erhöht, dass die Untereinheiten zu den Kapsoiden assemblieren. Auf welchen Wert die Ionenstärke dabei zu erhöhen ist, hängt von der Art der Untereinheiten ab. Für die bekannten Kapsoide bildenden Untereinheiten ist es bekannt, bei welcher Ionenstärke sich Kapsoide bilden. Da sich die Bildung der Kapsoide aber auch leicht durch eine Zunahme der Lichtstreuung verfolgen lässt, lässt sich die zur Auslösung der Kapsoid-Assemblierung erforderliche Erhöhung der Ionenstärke auch einfach ermitteln.

Unter Lösung im Sinne der Erfindung ist auch eine kolloide Lösung oder eine Suspension zu verstehen. Bei dem Reduktionsmittel kann es sich um Dithiothreitol (DTT), β-Mercaptoethanol, Glutathion, Dithioerythritol, Cystein, eine SH-Gruppe oder ein 2-Mercaptoethansulfonat-Natrium-Salz handeln. Um stabile Kapsoide zu erhalten, wird das Reduktionsmittel inaktiviert oder aus der Lösung entfernt. Dadurch können sich intra-kapsomere, insbesondere intra-pentamere, Disulfidbrücken ausbilden, die sich durch nachfolgende Konformationsänderungen stabilisierend auf die inter-kapsomeren, insbesondere inter-pentameren, Wechselwirkungen auswirken. Unter dem Inaktivieren des Reduktionsmittels wird verstanden, dass es so behandelt wird, dass es die Oxidation von in den Untereinheiten enthaltenen SH-Gruppen zu Disulfidbrücken durch ein in der Lösung enthaltenes Oxidationsmittel, wie z. B. darin gelösten Luftsauerstoff, nicht mehr verhindern kann. Das kann sehr schnell, z. B. durch einen spezifischen Abbau oder eine Oxidation des Reduktionsmittels erfolgen. Das Entfernen des Reduktionsmittels kann ebenfalls sehr schnell erfolgen, z. B. durch ein chromatografisches Verfahren, insbesondere mittels einer kommerziell erhältlichen Entsalzungssäule. Das Reduktionsmittel muss nur so weit entfernt werden, dass die Ausbildung von Disulfidbrücken durch Oxidation von in den Untereinheiten enthaltenen SH-Gruppen durch ein in der Lösung enthaltenes Oxidationsmittel, wie z. B. gelösten Sauerstoff, möglich ist. Ein quantitatives Entfernen des Reduktionsmittels ist dazu nicht erforderlich. Die Ionenstärke in der die Untereinheiten enthaltenden Lösung muss so niedrig sein, dass sich durch das bloße Inaktivieren oder Entfernen des Reduktionsmittels noch keine Kapsoide bilden. Das bedeutet, dass die Ionenstärke in der die Untereinheiten enthaltenden Lösung anfangs auch höher sein kann, wenn die dafür verantwortlichen Ionen zusammen mit dem Reduktionsmittel entfernt werden.

Das zur Erhöhung der Ionenstärke zur Lösung zuzusetzende Salz kann in fester Form oder als Lösung zugesetzt werden. Nach dem Zusetzen des Salzes sollte eine Inkubation der Lösung erfolgen, in der sich die Kapsoide bilden können. Vorzugsweise erfolgt die Inkubation bei Raumtemperatur für etwa 30 min.

Das gesamte Verfahren kann nach 1 bis 2 Stunden abgeschlossen sein. Überraschenderweise hat sich gezeigt, dass die dabei gebildeten Kapsoide trotz ihrer schnellen Bildung durch bloße Erhöhung der Ionenstärke von ebenso regelmäßiger Struktur sind, wie die durch das sehr langwierige Dialyseverfahren gewonnenen Kapsoide. Das Verfahren hat den Vorteil, dass die Bedingungen, unter denen das Assemblieren stattfindet, wie z. B. Ionenstärke, pH, Temperatur oder Proteinkonzentration, genauer vorgegeben und kontrolliert werden können als bei dem Dialyseverfahren. Im Hinblick auf die Anwendung des erfindungsgemäßen Verfahrens zur Verpackung eines Wirkstoffs in Kapsoide ist das von entscheidender Bedeutung. Beim oben beschriebenen Dialyseverfahren bilden sich die Kapsoide nämlich sobald eine bestimmte Salzkonzentration erreicht und eine bestimmte Konzentration des Reduktionsmittels unterschritten wird. Es ist jedoch nicht möglich, die Bildung der Kapsoide bei bestimmten vorgegebenen, beispielsweise für den darin zu verpackenden Wirkstoff günstigen, Bedingungen durchzuführen. Bei dem erfindungsgemäßen Verfahren können dagegen für den Wirkstoff günstige Bedingungen eingestellt werden, so weit dabei die Untereinheiten noch zu den Kapsoiden assemblieren.

Ein weiterer Vorteil des erfindungsgemäßen Verfahrens gegenüber dem Dialyseverfahren besteht darin, dass auch ein Wirkstoff in den Kapsoiden verpackt werden kann, der gegenüber dem Reduktionsmittel empfindlich ist, indem der Wirkstoff erst nach dem Entfernen oder Inaktivieren des Reduktionsmittels zugesetzt wird.

Weiterhin ist es bei dem Verfahren vorteilhaft, dass der Kontakt der Lösung und des darin gegebenenfalls enthaltenen Wirkstoffs mit einer Dialysemembran vermieden werden kann. An der Dialysemembran kann es ansonsten zu unspezifischen Bindungen kommen. Das würde zu einem Verlust an Wirkstoff und Untereinheiten führen. Weiterhin ist es mit dem Verfahren auch möglich, kleine Wirkstoff-Moleküle in Kapsoide zu verpacken, die bei dem Dialyseverfahren die Dialysemembran passieren würden. Damit solche Wirkstoff-Moleküle nicht zusammen mit dem Reduktionsmittel entfernt werden, werden sie der Lösung bevorzugt erst nach dem Entfernen des Reduktionsmittels zugesetzt.

Ein weiterer Vorteil des erfindungsgemäßen Verfahrens besteht darin, dass, schon wegen der geringeren Menge zu handhabender Flüssigkeiten, sterile Bedingungen leichter einzuhalten sind als bei dem Dialyseverfahren.

In einer bevorzugten Ausgestaltung des Verfahrens wird das Reduktionsmittel mittels einer Größenausschlusschromatografie oder einer Dialyse entfernt oder mittels eines Oxidationsmittels inaktiviert, welches im Wesentlichen nur das Reduktionsmittel oxidiert. Insbesondere wird das Oxidationsmittel so gewählt, dass dessen Redoxpotenzial nicht zur Oxidation der SH-Gruppen in den Untereinheiten ausreicht. Die folgenden Oxidations-Reduktionsmittel-Kombinationen haben sich dabei als besonders vorteilhaft erwiesen: oxidiertes Glutathion - reduziertes Glutathion, Cystin - Cystein, Cystamin - Cysteamin, Di(2-hydroxyethyl)-disulfid - β-Mercaptoethanol.

Die Größenausschlusschromatografie hat den Vorteil, dass sie sehr schnell durchzuführen ist. Ein weiterer Vorteil besteht darin, dass das Verfahren sehr gut von einen Laborverfahren in kleinem Maßstab auf ein Verfahren zur Produktion größerer Kapsoid-Mengen übertragen werden kann. Da bei dem erfindungsgemäßen Verfahren nur die im Allgemeinen niedermolekularen Reduktionsmittel entfernt werden müssen, ist dies jedoch auch durch eine Dialyse schnell durchzuführen.

Die Ionenstärke kann schrittweise erhöht werden. Dadurch kann die Homogenität der Kapsoide weiter gesteigert werden. Als günstig hat es sich erwiesen die Ionenstärke in 5 gleich großen Schritten zu erhöhen. Vorzugsweise beträgt der zeitliche Abstand zwischen den Schritten etwa 10 Minuten. Nach dem letzten Schritt ist eine ca. 10-minütige Inkubation bei Raumtemperatur vorteilhaft.

Die Ionenstärke I kann auf einen Wert von maximal 1,5 mol/l, insbesondere maximal 1 mol/l, vorzugsweise maximal 0,75 mol/l, erhöht werden. Dabei ist I = 0,5 * Σ cᵢ * zᵢ² , wobei cᵢ die Konzentration und zᵢ die Ladung der Ionen i in der Lösung ist.

Bevorzugt wird in der Lösung eine durch die Kapsoide und die Untereinheiten bedingte Gesamt-Proteinkonzentration von 15 µg/ml, insbesondere 150 µg/ml, vorzugsweise 225 µg/ml, nicht unterschritten. Dadurch kann beim Assemblieren eine höhere Ausbeute und Regelmäßigkeit der Kapsoide erreicht werden. Das Assemblieren erfolgt umso schneller, je höher die Konzentration der Untereinheiten in der Lösung ist.

Bei einer vorteilhaften Ausgestaltung des Verfahrens bestehen die Untereinheiten aus rekombinant hergestellten Proteinen oder Peptiden. Das ist insbesondere dann vorteilhaft, wenn große Mengen der Kapsoide hergestellt werden sollen. Es muss kein üblicherweise in Form von Kapsiden vorliegendes natives und möglicherweise infektiöses Ausgangsmaterial verwendet werden, welches vor dem Assemblieren erst deassembliert werden müsste. Vorzugsweise enthalten die Untereinheiten das Virusprotein "VP1" eines Polyoma-Viruses, das Virusprotein "L1" eines Papilloma-Viruses, das "Core-Protein" zusammen mit dem "Membrane-Protein" und dem "Envelope-Protein" des Flaviviruses, das "Core-Protein" des Hepatitis B-Viruses oder des Hepatitis C-Viruses, das Virusprotein "VP1" des SV40-Viruses, das Virusprotein "gag" des HI-Viruses, das Virusprotein "VP5" des Herpes Simplex Viruses, das Virusprotein "Lambda1", "Lambda2" oder "Lambda3" des Reoviruses oder das "Capsid-Protein" des Norwalk Viruses. Diese Virusproteine sind besonders gut zur Herstellung von Kapsoiden zur Verpackung von Wirkstoffen geeignet.

Bei einer vorteilhaften Ausgestaltung des Verfahrens werden in den Untereinheiten enthaltene SH-Gruppen nach dem Assemblieren zu den Kapsoiden oxidiert. Das bewirkt die Bildung intra-kapsomerer, insbesondere intra-pentamerer, Disulfidbrücken und wegen der dadurch ausgelösten Konformationsänderung eine Stabilisierung der Kapsoide. Die Bedingungen werden dabei vorteilhafterweise so gewählt, dass es nicht zur Ausbildung von Disulfidbrücken zwischen den Kapsomeren, insbesondere Pentameren, kommt. Besonders vorteilhaft ist es, wenn die SH-Gruppen durch Zusatz eines Oxidationsmittels, insbesondere Cystin, Cystamin, Di(2-hydroxyethyl)-disulfid oder oxidiertem Glutathion (GSSG), oxidiert werden. Als günstig hat sich eine Inkubation mit etwa 7 mmol/l oxidiertem Glutathion für etwa 30 Minuten bei Raumtemperatur erwiesen. Danach kann das Oxidationsmittel, z. B. durch Dialyse oder ein chromatografisches Verfahren, entfernt oder durch Zusatz eines Reduktionsmittels inaktiviert werden.

Die Lösung kann bei der Erhöhung der Ionenstärke einen Wirkstoff enthalten. Dabei können sich den Wirkstoff enthaltende Kapsoide bilden, welche als Vehikel dienen können, um den Wirkstoff in Zellen einzuschleusen. Der Wirkstoff kann der Lösung auch erst zugesetzt werden, nachdem das Reduktionsmittel entfernt oder inaktiviert worden ist. Das ist dann besonders vorteilhaft, wenn der Wirkstoff gegenüber dem Reduktionsmittel empfindlich ist. Bei dem Wirkstoff kann es sich um einen im Inneren von Zellen wirkenden Stoff, insbesondere eine Nukleinsäure, ein Protein, einen Antikörper, ein Peptid, ein Enzym, einen Transkriptionsfaktor, ein phosphorothioatderivatisiertes Oligonukleotid, PNA, ein Chimär aus PNA und DNA, ein DNA-Peptid-Komplex oder einen niedermolekularen Wirkstoff, handeln. Der Wirkstoff kann mit mindestens einer der Untereinheiten gekoppelt oder assoziiert werden. Das ermöglicht den gezielten Transport des Wirkstoffs in einen vorgegebenen Bereich innerhalb einer eukaryotischen Zelle. Verfahren zum Koppeln oder Assoziieren sind aus der WO 00/00224 bekannt. Vorzugsweise wird der Wirkstoff derart mit der Untereinheit gekoppelt oder assoziiert, dass er nach dem Assemblieren auf der Innenseite der Kapsoide lokalisiert ist. Der Einschluss des Wirkstoffs in die Kapsoide bewirkt eine verbesserte Aufnahme des Wirkstoffs in die Zelle.

Bei einer Ausgestaltung des Verfahrens werden die assemblierten Kapsoide lyophilisiert. Das ist dann besonders günstig, wenn in den Kapsoiden ein Wirkstoff enthalten ist, welcher in gelöster Form mit der Zeit abgebaut werden oder in sonstiger Weise seine Wirksamkeit verlieren kann. Da auch das Verpacken in die Kapsoide sehr viel schneller möglich ist als beim Dialyseverfahren, kann durch das Lyophilisieren erfindungsgemäß hergestellter Kapsoide auch der Abbau oder der Wirksamkeitsverlust eines solchen Wirkstoffs früher und damit besser verhindert werden als bei Kapsoiden, welche mit dem herkömmlichen Verfahren hergestellt worden sind.

Die Erfindung betrifft ferner einen Kit zur Durchführung des erfindungsgemäßen Verfahrens, enthaltend
- Kapsoide bildende Untereinheiten in einer ein Reduktionsmittel enthaltenden Lösung und
- ein zum Inaktivieren des Reduktionsmittels geeignetes Oxidationsmittel, das im Wesentlichen nur das Reduktionsmittel oxidiert.

Der Kit kann auch ein Salz zur Erhöhung der Ionenstärke enthalten. Das Oxidationsmittel und das Salz können in einer vorbestimmten Menge und/oder gelöst in einer vorbestimmten Konzentration enthalten sein.

Ausführungsbeispiele der Erfindung sind in der Zeichnung dargestellt und werden in der nachfolgenden Beschreibung näher erläutert. Es zeigen:
- Fig. 1: eine schematische Darstellung eines erfindungsgemä- ßen Verfahrens,
- Fig. 2a,b: je eine elektronenmikroskopische Aufnahme von nach dem erfindungsgemäßen Verfahren und nach dem her- kömmlichen Verfahren hergestellten Kapsoiden aus dem Virusprotein VP1,
- Fig. 3: grafische Darstellung einer durch erfindungsgemäß hergestellte Kapsoide verursachten Lichtstreuung in Abhängigkeit von der Zeit nach Zusatz des Salzes,
- Fig. 4a,b: grafische Darstellung je einer Analyse der in der Lösung enthaltenen Partikel vor und nach Zugabe des Salzes mittels Photonenkorrelationsspektroskopie (PCS) und
- Fig. 5a,b: grafische Darstellung je einer analytischen Gelfil- tration der in der Lösung enthaltenen Partikel vor und nach Zugabe des Salzes.

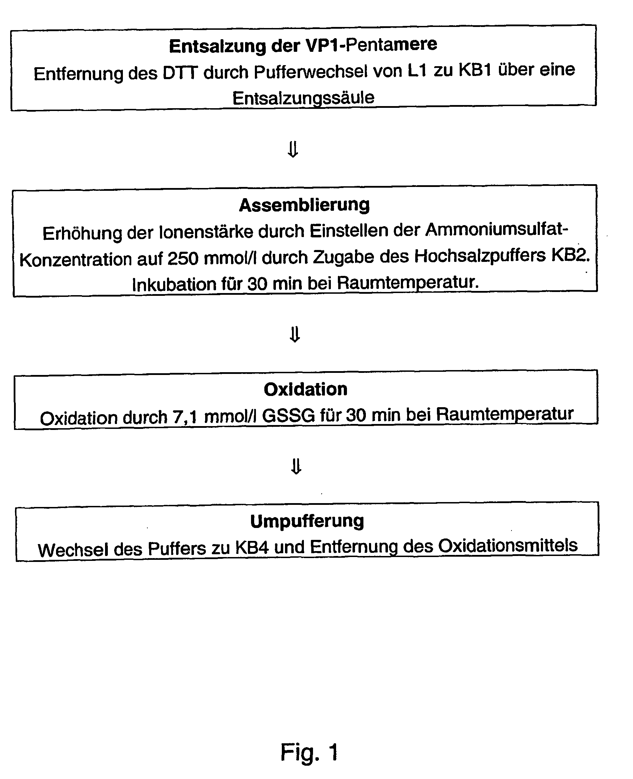

Das Hüllprotein VP1 des Polyoma-Virus wird rekombinant als Homopentamer aus *E. coli* gewonnen und chromatografisch gereinigt. Am Ende der Reinigung liegt es im so genannten L1-Puffer (50 mmol/l Na-Phosphat, 150 mmol/l NaCl, 2 mmol/l EDTA, 5 % Glycerin, 6 mmol/l DTT, pH 7,0) unter reduzierenden Bedingungen vor. In Fig. 1 ist schematisch der Ablauf des damit durchgeführten erfindungsgemäßen Verfahrens dargestellt. Um durch das erfindungsgemäße Verfahren stabile VP1-Kapsoide - es werden intra-pentamere Disulfidbrücken ausgebildet, die sich durch nachfolgende Konformationsänderungen stabilisierend auf die inter-pentamer Wechselwirkungen auswirken - zu erhalten, ist es notwendig, zuvor das Reduktionsmittel DTT aus der VP1-Pentamer-Lösung zu entfernen. Dieses geschieht mit Hilfe einer kommerziell erhältlichen Entsalzungssäule, wie "PD10" für analytische oder "HiPrep 26/10 Desalting" für präparative Ansätze, welche beide von der Firma Amersham Biosciences erhältlich sind. Als mobile Phase wird 50 mmol/l Na-Phosphat, 150 mmol/l NaCl, 2 mmol/l EDTA, 5 % Glycerin, pH 6,8 (im Weiteren als KB1-Puffer bezeichnet) verwendet. Das Protein befindet sich wegen der geringen Porengröße des Säulenmaterials im Durchlauf, während das Reduktionsmittel auf Grund seiner geringeren Größe verzögert von der Säule eluiert. Das Protein wird durch diese Prozedur leicht verdünnt. Die Konzentration an VP1 sollte 250 µg/ml nicht unterschreiten. Alternativ zu dem beschriebenen Verfahren kann das Reduktionsmittel auch mittels Dialyse gegen den KB1-Puffer entfernt werden.

Das Assemblieren der VP1-Pentamere wird durch Zugabe eines Hochsalzpuffers eingeleitet. Dafür muss das eingesetzte Volumen der VP1-Lösung bekannt sein, um die exakte Ionenstärke für das Assemblieren einstellen zu können. Der Assemblierungspuffer KB2 (10 mmol/l Tris/HCl, 150 mmol/l NaCl, 5 % Glycerin, 3 mol/l Ammomiumsulfat, pH 8,0) wird dann im Verhältnis 1:12 (1 Teil KB2, 11 Teile Proteinlösung) in die Proteinlösung hineinverdünnt und gemischt. Die Endkonzentration an Ammoniumsulfat beträgt nach diesem Schritt 250 mmol/l. Das entspricht einer Ionenstärke von 750 mmol/l. Es schließt sich eine Inkubationsphase von 30 Minuten bei Raumtemperatur an, in der die Kapsoidbildung vollständig abläuft. Um die Homogenität der Kapsoide weiter zu steigern, kann der Assemblierungsschritt in der Hinsicht modifiziert werden, dass der Hochsalzpuffer in mehreren Schritten der Froteinlösung zugesetzt wird. Mit fünf gleich großen Schritten im Abstand von 10 Minuten wird die Ammoniumsulfat-Konzentration schrittweise auf 250 mmol/l erhöht. Diese Prozedur wird dann mit einer 10-minütigen Inkubation bei Raumtemperatur abgeschlossen.

Die gewonnenen VP1-Kapsoide werden zur Stabilisierung mittels oxidiertem Glutathion oxidativen Bedingungen ausgesetzt. Dadurch kommt es zur Ausbildung intra-pentamerer Disulfidbrükken. Dazu wird die Proteinlösung mit dem KB3-Puffer (10 mmol/l Tris/HCl, 150 mmol/l NaCl, 5 % Glycerin, 3 mol/l Ammoniumsulfat, 400 mmol/l GSSG, pH 8,0) im Verhältnis 1:56 (1 Teil KB3, 55 Teile Proteinlösung; GSSG-Endkonzentration 7,1 mmol/l) versetzt und anschließend 30 Minuten bei Raumtemperatur inkubiert.

Gebildete Kapsoide werden im letzten Schritt gegen PBS-Puffer (PBS Dublecco, Biochrom) mit 0,7 mmol/l CaCl₂ (KB4) dialysiert. Dabei wird das Oxidationsmittel entfernt. Das Protein kann über Diafiltration oder Fällung auf die gewünschte Konzentration aufkonzentriert werden.

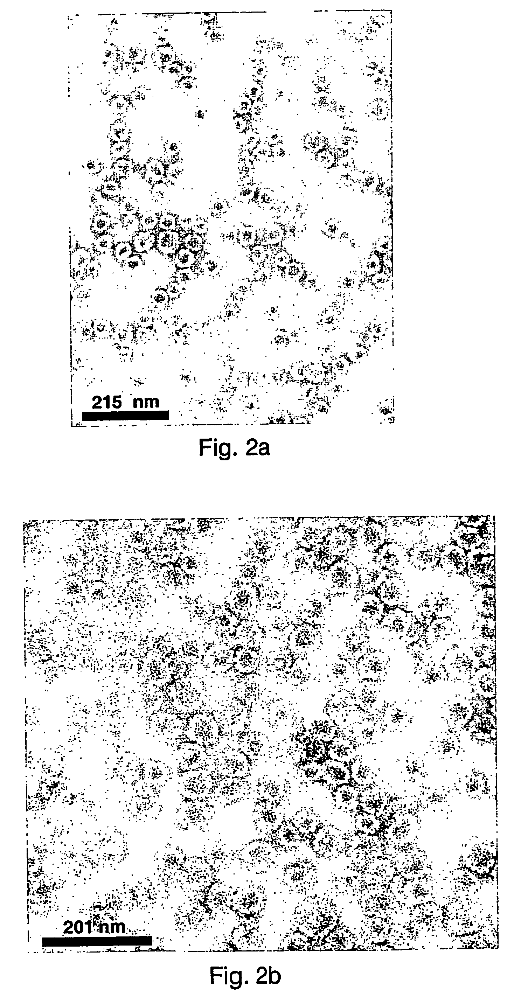

Die Figuren 2a und 2b zeigen jeweils eine elektronenmikroskopische Aufnahme von VP1-Kapsoiden. Die in Fig. 2a gezeigten Kapsoide sind mittels des erfindungsgemäßen Verfahrens hergestellt worden. Die in Fig. 2b gezeigten Kapsoide sind mittels des herkömmlichen Verfahrens, bei dem die Ionenstärke durch Dialyse unter gleichzeitiger Entfernung des Reduktionsmittels erhöht wird, gebildet worden. Die Figuren 2a und 2b zeigen, dass die mit dem erfindungsgemäßen Verfahren hergestellten Kapsoide von gleicher Qualität wie die herkömmlich hergestellten Kapsoide sind. Sie sind regelmäßig geformt und weisen einen Durchmesser von etwa 40 nm auf.

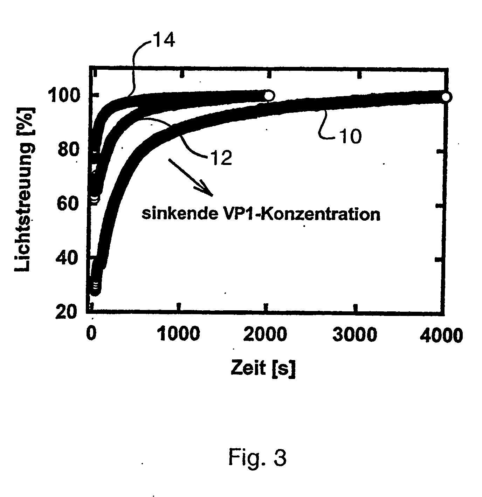

Die Kapsoide streuen wegen ihres gegenüber den Pentameren größeren Durchmessers in die Lösung eingestrahltes Licht stärker als die Pentamere. Das Ausmaß der Lichtstreuung der Lösung kann daher als Maß für den Gehalt der Lösung an Kapsoiden verwendet werden, so dass damit der Verlauf der Bildung der Kapsoide verfolgt werden kann. Fig. 3 zeigt die grafische Darstellung einer durch erfindungsgemäß hergestellte Kapsoide verursachten Lichtstreuung in Abhängigkeit von der Zeit nach Zusatz des Salzes. Die Kurven 10, 12 und 14 entsprechen den VP1-Konzentrationen 194 µg/ml, 387 µg/ml und 775 µg/ml. Daraus ist ersichtlich, dass die Bildung der Kapsoide umso schneller erfolgt, je höher die VP1-Konzentration ist. Sie kann bei hoher VP1-Konzentration bereits nach wenigen Minuten abgeschlossen sein.

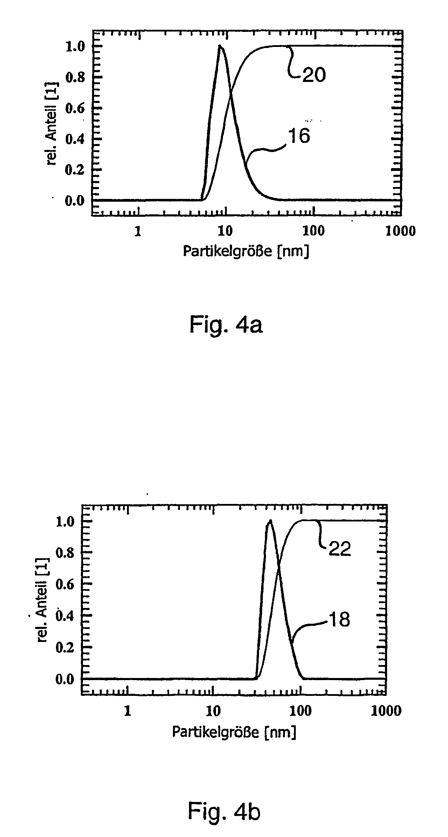

Die Größe der gebildeten Partikel kann auch mittels der Photonenkorrelationsspektroskopie (PCS) ermittelt werden. Dabei wird die Tatsache ausgenutzt, dass sich Partikel in einer Lösung umso langsamer bewegen, je größer sie sind. In den Figuren 4a und 4b zeigen die Kurven 16 und 18 jeweils den mittels der PCS bestimmten relativen Anteil der Partikel einer bestimmten Größe an der Gesamtzahl der Partikel, wobei die Einheiten willkürlich gewählt sind. Die Kurven 20 und 22 sind jeweils Integrationskurven der Kurven 16 und 18. Fig. 4a zeigt das Ergebnis einer vor dem Assemblieren durchgeführten PCS-Messung. Der Peak der Kurve 16 resultiert aus den Pentameren mit einem Durchmesser von etwa 10 nm. In Fig. 4b ist das Ergebnis einer PCS-Messung nach dem Assemblieren der Pentamere dargestellt. Der Peak der Kurve 18 wird durch die gebildeten Kapsoide mit einem Durchmesser von etwa 40 nm verursacht. Er ist gegenüber dem Peak der Kurve 16 aus Fig. 4a deutlich verschoben. Der Vergleich der Kurven 16 und 18 zeigt, dass die Pentamere vollständig zu Kapsoiden assembliert sind. Aus dem für eine Messung mittels PCS verhältnismäßig schmalen Peak der Kurve 18 geht außerdem hervor, dass die Kapsoide von einheitlicher Größe sind.

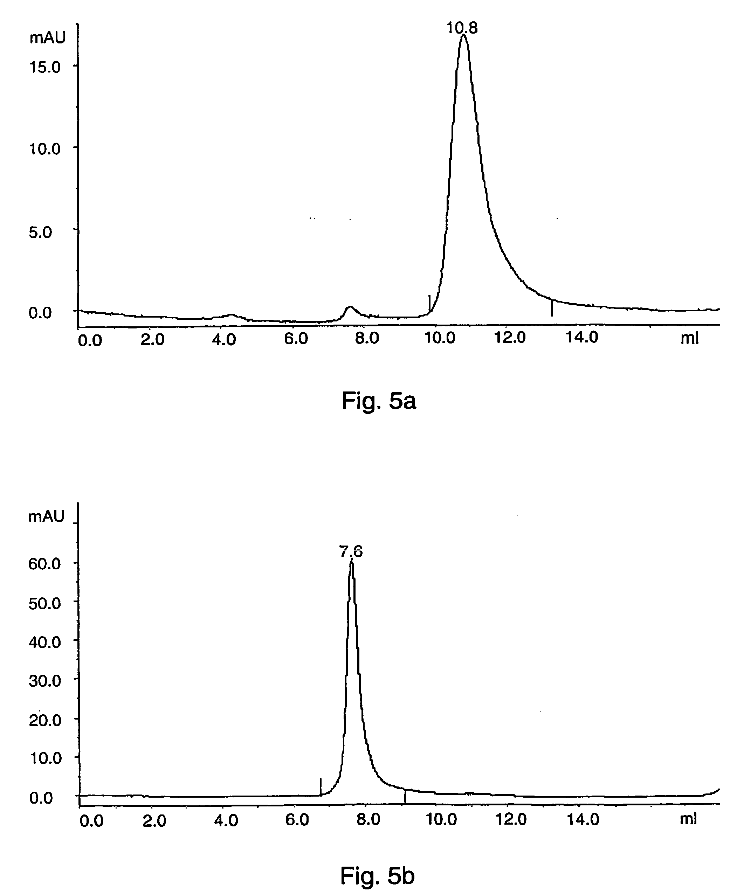

Weiterhin ist eine analytische Gelfiltration durchgeführt worden. Der Proteingehalt des Eluats ist durch Messung der UV-Absorption bei einer Wellenlänge von 280 nm ermittelt worden. Fig. 5a zeigt das für VP1-Pentamere und Fig. 5b das für die daraus gebildeten Kapsoide ermittelte Ergebnis, wobei für die Absorption jeweils willkürliche Einheiten (mAU) gewählt worden sind. Die VP1-Pentamere erzeugen einen Peak bei einem Retentionsvolumen von 10,82 ml. Dieser Peak ist in Fig. 5b nicht mehr zu sehen. Dem ist zu entnehmen, dass die VP1-Pentamere vollständig zu Kapsoiden assembliert sind. Der schmale bei einem Retentionsvolumen von 7,66 ml erscheinende durch die gebildeten Kapsoide verursachte Peak in Fig. 5b zeigt an, dass die Kapsoide von einheitlicher Größe sind.

## Patentansprüche

1. Verfahren zum Assemblieren von Kapsoide bildenden Untereinheiten in einer ein Reduktionsmittel enthaltenden Lösung zu Kapsoiden, wobei zuerst das Reduktionsmittel inaktiviert oder aus der Lösung entfernt wird und dann die Ionenstärke in der Lösung durch Zusatz mindestens eines Salzes zu der Lösung zumindest so weit erhöht wird, dass die Untereinheiten zu den Kapsoiden assemblieren.

2. Verfahren nach Anspruch 1, wobei das Reduktionsmittel mittels einer Größenausschlusschromatografie oder einer Dialyse entfernt oder mittels eines im Wesentlichen nur das Reduktionsmittel oxidierenden Oxidationsmittels inaktiviert wird.

3. Verfahren nach Anspruch 1 oder 2, wobei die Erhöhung der Ionenstärke in, vorzugsweise 5 gleich großen, Schritten erfolgt.

4. Verfahren nach Anspruch 3, wobei zwischen den Schritten ein zeitlicher Abstand von etwa 10 Minuten liegt.

5. Verfahren nach einem der vorhergehenden Ansprüche, wobei die Ionenstärke I auf einen Wert von maximal 1,5 mol/l, insbesondere maximal 1 mol/l, vorzugsweise maximal 0,75 mol/l, erhöht wird.

6. Verfahren nach einem der vorhergehenden Ansprüche, wobei während des Verfahrens in der Lösung eine durch die Kapsoide und die Untereinheiten bedingte Gesamt-Proteinkonzentration von 15 µg/ml, insbesondere 150 µg/ml, vorzugsweise 225 µg/ml, nicht unterschritten wird.

7. Verfahren nach einem der vorhergehenden Ansprüche, wobei die Untereinheiten aus rekombinant hergestellten Proteinen oder Peptiden bestehen.

8. Verfahren nach einem der vorhergehenden Ansprüche, wobei die Untereinheiten das Virusprotein "VP1" eines Polyoma-Viruses, das Virusprotein "L1" eines Papilloma-Viruses, das "Core-Protein" zusammen mit dem "Membrane-Protein" und dem "Envelope-Protein" des Flaviviruses, das "Core-Protein" des Hepatitis B-Viruses oder des Hepatitis C-Viruses, das Virusprotein "VP1" des SV40-Viruses, das Virusprotein "gag" des HI-Viruses, das Virusprotein "VP5" des Herpes Simplex Viruses, das Virusprotein "Lambda1", "Lambda2" oder "Lambda3" des Reoviruses oder das "Capsid-Protein" des Norwalk Viruses enthalten.

9. Verfahren nach einem der vorhergehenden Ansprüche, wobei in den Untereinheiten enthaltene SH-Gruppen nach dem Assemblieren zu den Kapsoiden oxidiert werden.

10. Verfahren nach Anspruch 9, wobei die SH-Gruppen durch Zusatz eines Oxidationsmittels, insbesondere Cystin, Cystamin, Di(2-hydroxyethyl)-disulfid oder oxidiertem Glutathion, oxidiert werden.

11. Verfahren nach einem der vorhergehenden Ansprüche, wobei die Lösung bei der Erhöhung der Ionenstärke einen Wirkstoff enthält.

12. Verfahren nach Anspruch 11, wobei der Wirkstoff der Lösung erst zugesetzt wird nachdem das Reduktionsmittel entfernt oder inaktiviert worden ist.

13. Verfahren nach Anspruch 11 oder 12, wobei der Wirkstoff ein im Inneren von Zellen wirkender Stoff, insbesondere eine Nukleinsäure, ein Protein, ein Antikörper, ein Peptid, ein Enzym, ein Transkriptionsfaktor, ein phosphorothioatderivatisiertes Oligonukleotid, PNA, ein Chimär aus PNA und DNA, ein DNA-Peptid-Komplex oder ein niedermolekularer Wirkstoff, ist.

14. Verfahren nach einem der Ansprüche 11 bis 13, wobei der Wirkstoff mit mindestens einer der Untereinheiten gekoppelt oder assoziiert wird.

15. Verfahren nach Anspruch 14, wobei der Wirkstoff derart mit der Untereinheit gekoppelt oder assoziiert wird, dass er nach dem Assemblieren auf der Innenseite der Kapsoide lokalisiert ist.

16. Verfahren nach einem der vorhergehenden Ansprüche, wobei die Kapsoide lyophilisiert werden.

17. Kit zur Durchführung eines Verfahrens nach einem der vorhergehenden Ansprüche, enthaltend
- Kapsoide bildende Untereinheiten in einer ein Reduktionsmittel enthaltenden Lösung,
- ein zum Inaktivieren des Reduktionsmittels geeignetes Oxidationsmittel, das im Wesentlichen nur das Reduktionsmittel oxidiert und
- ein Salz zur Erhöhung der Ionenstärke.

18. Kit nach Anspruch 17, wobei das Oxidationsmittel in einer vorbestimmten Menge und/oder gelöst in einer vorbestimmten Konzentration enthalten ist.

19. Kit nach Anspruch 17 oder 18, wobei das Salz in einer vorbestimmten Menge und/oder gelöst in einer vorbestimmten Konzentration enthalten ist.

## Claims

1. A method for assembling capsoid-forming subunits in a solution containing a reducing agent to give capsoids, in which method the reducing agent is initially inactivated or removed from the solution and the ionic strength in said solution is then increased, by adding at least one salt to said solution, to at least such an extent that said subunits assemble to give said capsoids.

2. The method as claimed in claim 1, in which the reducing agent is removed by means of size exclusion chromatography or dialysis or is inactivated by means of an oxidizing agent which oxidizes essentially only said reducing agent.

3. The method as claimed in claim 1 or 2, in which the ionic strength is increased in steps that are preferably 5 equally large steps.

4. The method as claimed in claim 3, in which there is a time interval of about 10 minutes between the steps.

5. The method as claimed in any of the preceding claims, in which the ionic strength I is increased to a value of no more than 1.5 mol/l, in particular no more than 1 mol/l, preferably no more than 0.75 mol/l.

6. The method as claimed in any of the preceding claims, during which the total protein concentration in the solution, due to the capsoids and the subunits, does not fall below 15 µg/ml, in particular 150 µg/ml, preferably 225 µg/ml.

7. The method as claimed in any of the preceding claims, in which the subunits consist of recombinantly produced proteins or peptides.

8. The method as claimed in any of the preceding claims, in which the subunits comprise the viral protein "VP1" of a polyoma virus, the viral protein "L1" of a papilloma virus, the "core protein", together with the "membrane protein" and the "envelope protein", of the flavi virus, the "core protein" of the hepatitis B virus or of the hepatitis C virus, the viral protein "VP1" of the SV40 virus, the viral protein "gag" of the HI virus, the viral protein "VP5" of the herpes simplex virus, the viral protein "lambdal", "lambda2" or "lambda3" of the reo virus or the "capsid protein" of the Norwalk virus.

9. The method as claimed in any of the preceding claims, in which SH groups present in the subunits are oxidized after the assembly to give the capsoids.

10. The method as claimed in claim 9, in which the SH groups are oxidized by adding an oxidizing agent, in particular cystine, cystamine, di(2-hydroxyethyl) disulfide or oxidized glutathione.

11. The method as claimed in any of the preceding claims, in which the solution comprises an active compound when the ionic strength is increased.

12. The method as claimed in claim 11, in which the active compound is added to the solution only after the reducing agent has been removed or inactivated.

13. The method as claimed in claim 11 or 12, in which the active compound is a substance acting inside cells, in particular a nucleic acid, a protein, an antibody, a peptide, an enzyme, a transcription factor, a phosphorothioate-derivatized oligonucleotide, PNA, a chimera of PNA and DNA, a DNA-peptide complex or a low molecular weight active compound.

14. The method as claimed in any of claims 11 to 13, in which the active compound is coupled to or associated with at least one of the subunits.

15. The method as claimed in claim 14, in which the active compound is coupled to or associated with the subunit in such a way that, after the assembly, it is located on the inside of the capsoids.

16. The method as claimed in any of the preceding claims, in which the capsoids are lyophilized.

17. A kit for carrying out a method as claimed in any of the preceding claims, which kit comprises
- capsoid-forming subunits in a solution containing a reducing agent,
- an oxidizing agent suitable for inactivating the reducing agent, which oxidizes essentially only said reducing agent and
- a salt for increasing the ionic strength.

18. The kit as claimed in claim 17, in which the oxidizing agent is present in a predetermined amount and/or dissolved at a predetermined concentration.

19. The kit as claimed in claim 17 or 18, in which the salt is present in a predetermined amount and/or dissolved at a predetermined concentration.

## Revendications

1. Procédé d'assemblage en capsoides de sous-unités formant des capsoides dans une solution contenant un agent réducteur, dans lequel l'agent réducteur est d'abord inactivé ou enlevé de la solution, puis on augmente la force ionique dans la solution par ajout d'au moins un sel à la solution au moins dans une mesure telle que les sous-unités s'assemblent en capsoides.

2. Procédé selon la revendication 1, dans lequel l'agent réducteur est enlevé au moyen d'une chromatographie d'exclusion de taille ou d'une dialyse ou inactivé au moyen d'un agent oxydant qui oxyde essentiellement uniquement l'agent réducteur.

3. Procédé selon la revendication 1 ou 2, dans lequel l'augmentation de la force ionique se fait par étapes, de préférence en 5 étapes de même taille.

4. Procédé selon la revendication 3, dans lequel il existe un intervalle d'environ 10 minutes entre les étapes.

5. Procédé selon l'une des revendications précédentes, dans lequel la force ionique I est augmentée à une valeur au maximum égale à 1,5 mol/l, en particulier au maximum égale à 1 mol/l, de préférence au maximum égale à 0,75 mol/l.

6. Procédé selon l'une des revendications précédentes, dans lequel pendant le procédé, dans la solution, on ne passe pas sous une concentration totale en protéines conditionnée par les sous-unités et les capsoides de 15 µg/ml, en particulier de 150 µg/ml, de préférence de 225 µg/ml.

7. Procédé selon l'une des revendications précédentes, dans lequel les sous-unités se composent de protéines ou de peptides élaborés par voie recombinante.

8. Procédé selon l'une des revendications précédentes, dans lequel les sous-unités contiennent la protéine virale "VP1" d'un polyomavirus, la protéine virale "L1" d'un papillomavirus, la "protéine de noyau" avec la "protéine de membrane" et la "protéine d'enveloppe" du flavivirus, la "protéine de noyau" du virus de l'hépatite B ou du virus de l'hépatite C, la protéine virale "VP1" du virus SV40, la protéine virale "gag" du VIH, la protéine virale "VP5" de l'herpes simplex virus, la protéine virale "lambdal", "lambda2" ou "lambda3" du réovirus ou la "protéine de capside" du virus Norwalk.

9. Procédé selon l'une des revendications précédentes, dans lequel les groupes SH contenus dans les sous-unités sont oxydés après l'assemblage en capsoides.

10. Procédé selon la revendication 9, dans lequel les groupes SH sont oxydés par ajout d'un agent oxydant, en particulier la cystine, la cystamine, le di(2-hydroxyéthyl)-disulfure ou un glutathion oxydé.

11. Procédé selon l'une des revendications précédentes, dans lequel la solution, lors de l'augmentation de la force ionique, comprend un principe actif.

12. Procédé selon la revendication 11, dans lequel le principe actif est seulement ajouté à la solution après que l'agent réducteur ait été enlevé ou inactivé.

13. Procédé selon la revendication 11 ou 12, dans lequel le principe actif est une substance agissant à l'intérieur des cellules, en particulier un acide nucléique, une protéine, un anticorps, un peptide, une enzyme, un facteur de transcription, un oligonucléotide dérivé par phosphorothioate, un ANP, un chimère d'ANP et D'ADN, un complexe ADN-peptide ou un principe actif de faible poids moléculaire.

14. Procédé selon l'une quelconque des revendications 11 à 13, dans lequel le principe actif est couplé ou associé à au moins une des sous-unités.

15. Procédé selon la revendication 14, dans lequel le principe actif est couplé ou associé à la sous-unité de façon telle qu'il est localisé après l'assemblage sur le côté interne des capsoides.

16. Procédé selon l'une quelconque des revendications précédentes, dans lequel les capsoides sont lyophilisées.

17. Trousse pour la réalisation d'un procédé selon l'une des revendications précédentes, contenant
- des sous-unités formant des capsoides dans une solution contenant un agent réducteur,
- un agent oxydant approprié pour inactiver l'agent réducteur, qui oxyde essentiellement uniquement l'agent réducteur, et
- un sel pour l'augmentation de la force ionique.

18. Trousse selon la revendication 17, dans laquelle l'agent oxydant est contenu dans une quantité prédéfinie et/ou dissous selon une concentration prédéfinie.

19. Trousse selon la revendication 17 ou 18, dans laquelle le sel est contenu dans une quantité prédéfinie et/ou dissous selon une concentration prédéfinie.
